(19) Europäisches Patentamt
European Patent Office
Office européen des brevets

(11) Publication number: **0 046 971**
**A1**

(12) # EUROPEAN PATENT APPLICATION

(21) Application number: 81106583.8

(22) Date of filing: 25.08.81

(51) Int. Cl.[3]: A 61 M 1/03
B 01 J 39/02

(30) Priority: 27.08.80 US 181883

(43) Date of publication of application: 10.03.82 Bulletin 82/10

(84) Designated Contracting States: BE DE FR GB IT NL

(71) Applicant: UNION CARBIDE CORPORATION
270, Park Avenue
New York, N.Y. 10017(US)

(72) Inventor: Sherman, John Delano
91 Valley View Road
Chappaqua, N.Y. 10514(US)

(74) Representative: Eggert, Hans-Gunther, Dr.
Raederscheidtstrasse 1
D-5000 Köln 41(DE)

(54) **Removal of uremic substances with zeolite ion-exchangers.**

(57) Compositions of zeolite W are used in hemodialysis to extract ammonia and to regulate concentrations of calcium and potassium in the dialysate, with zeolite W being employed in the form of a slurry in the dialysate.

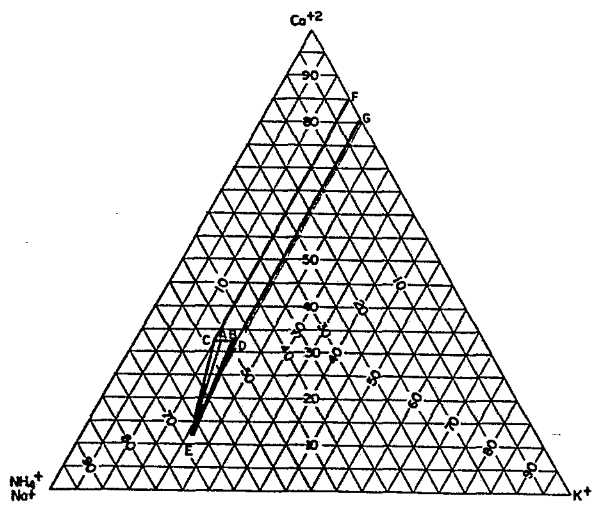


FIG. I

EP 0 046 971 A1

This invention relates to a process for removing uremic substances from the blood. In particular, it relates to the use of particular zeolite compositions as ion exchange media in a dialysate in a hemodialysis process.

Hemodialysis is a process for removing impurities from the blood in persons whose kidneys have failed. The principal impurity to be removed is urea. In a typical application, blood is withdrawn from the body, contacted with a semipermeable membrane and returned to the body. The side of the membrane opposite the blood is contacted by a solution called dialysate. The membrane excludes the passage of molecules on the basis of size. Therfore, molecules such as urea and ammonia and cations such as calcium, potassium, hydrogen and sodium can pass through the membrane, whereas larger molecules such as blood proteins are retained in the blood. In order for urea to pass from the blood, the concentration of urea in the dialysate must be lower. Also, body levels of cations, including potassium and calcium, must be regulated. This regulation is accomplished by maintaining a constant, predetermined concentration of these cations in the dialysate. At these predetermined levels removal or addition of the cation from the body will be within the desired levels. An often-used method to maintain a low urea concentration and the proper cation concentrations in the dialysate is to continuously pass fresh dialysate of specified composition over the membrane. Since the dialysate is used only once, this method requires preparation and treatment of large amounts of water. This requires bulky and heavy apparatus.

It has been proposed, therefore, to recycle the dialysate by continuously regenerating and reusing it. This would require much smaller amounts of water and also reduce the bulk of the dialysis apparatus.

To maintain a low urea concentration in the dialysate, for a recycle method the urea must either be sorbed or consumed. This can be accomplished by hydrolyzing the urea with the enzyme urease in accordance with the following formula:

(1) $$\underset{\text{Urea}}{(H_2N)_2CO} + 2H_2O \xrightarrow{\text{Urease}} 2NH_4^{+}\ CO_3^{-2}$$

The ammonium cations can then be removed with sorbent or ion-exchange medium that is selective towards ammonia. Although the ammonium cations exist primarily in the ionized ($NH_4^{+}$) form, for purposes of this specification, "ammonium", "ammonia" or "ammonium cation" are synonymous.

The carbonate is removed through reaction with calcium cations present in the dialysate according to the formula:

(2) $$CO_3^{-2} + Ca^{+2} \longrightarrow CaCO_3(s)$$

In addition, a minor amount of the carbonate produced will form an equilibrium with bicarbonate and also react with the calcium in the dialysate to form an equilibrium with calcium bicarbonate complex according to the equations:

(3) $$H^{+} + CO_3^{-2} \rightleftharpoons HCO_3^{-1}$$

(4) $$Ca^{+2} + H^{+} + CO_3^{-2} \rightleftharpoons CaHCO_3^{+}$$

The major portion of the carbonate from equation (1) is consumed in equation (2) and withdrawn from the dialysate

in the insoluble calcium carbonate product of equation (2). Calcium is also removed from the dialysate solution through reaction (2). If this calcium so removed is not replaced, the result will be depletion of calcium in the patient, leading to varied medical problems. Since substantially all of the carbonate is consumed in equation (2), the depletion of calcium will nearly be equal to the amount of carbonate produced in equation (1). Thus it can be seen by combining equations (1) and (2) that to maintain the calcium level, one milli-mole of calcium cations must be released into the dialysate for every mille-mole of urea consumed. Or in other terms, one milli-equivalent (meq) of calcium must be released for every milli-equivalent of carbonate produced.

Another impurity to be removed is potassium cations. Potassium levels that are too high can lead to paralysis of the muscles and possible death of the patient. On the other hand, if too much potassium is removed and the potassium levels in the body become too low, paralysis will likewise result.

In an average person approximately 11o meq per day of potassium need to be removed. This compares with approximately 6oo meq per day of urea (as ammonium carbonate) that needs to be removed. These figures will vary according to diet and other individual factors. However, for an average person the ratio of urea removed to potassium removed varies from about 5 to up to about 12 meq of urea removed (as ammonium carbonate) to 1 meq of potassium removed, or in terms of calcium release, 5 to 12 meq of calcium released to 1 meq of potassium removed. The above range is for average patients and is, therefore, the preferred range. The ratio appropriate for a particular patient will depend in part on the

potassium intake in the diet and the level of calcium in the body. In extreme cases where it may be necessary to replenish calcium in a patient or where there has been an excessive potassium intake in the diet or where for any reason the levels of potassium and calcium become abnormal, the ratio may be as low as 4 to as high as 16 meq of calcium released to 1 meq of potassium removed.

The sorbent or ion-exchanger used to remove the ammonia can be in the form of a fixed bed as is disclosed in British Patent Specification 1 484 642 issued to Gambro AG. A difficulty with this system is that the calcium carbonate precipitate created in reaction (2) tends to clog the bed. This reduces the efficiency of the bed and leads to undesirably high pressure-drops across the bed.

In U.S. Patent No. 4 o71 444, Ash et al. describe a reciprocating parallel plate dialyzer with chambers surrounded by a suspension of sorbent compounds. The suspension consists of solid sorbent particles evenly suspended as a slurry in the dialysate solution. A slurried system such as this would not have the calcium carbonate clogging problems inherent in a fixed-bed system. A slurry could also be advantageously used with other dialyzers to avoid the fixed-bed of adsorbents.

The slurry system differs from the fixed-bed system in that the sorbents, the dissolved components and other components of the slurry are more or less evenly distributed throughout the dialysate. Thus, unlike a fixed-bed system, the concentration of ammonia would be approximately the same near the semipermeable membrane as elsewhere in the dialysate. This will result in some

ammonia diffusing back through the membrane into the blood. Ammonia can become toxic if its blood concentration becomes excessive. Therefore, to prevent back-diffusion of excessive amounts of ammonia, the ammonia concentration of the dialysate must be kept at a low level, preferably below 3 milli-equivalents per liter (meq/l).

However, most adsorbents selective to ammonia have poor capacity for ammonia at the low concentrations that would be encountered in a slurried dialysate. Also, some adsorbents exhibit preference for other cations in the dialysate, thus lowering the available capacity for ammonia.

It is, therefore, an object of the invention to provide for a composition which has high capacity for ammonia at low concentrations of ammonia in the presence of other cations such that it can be used as in a slurried dialysate.

It is also an object of the invention to provide for a composition that releases calcium and removes potassium in the proper proportions.

Other objects will become apparent as the invention is described below.

The objects of the invention are accomplished by the use of a form of zeolite W. Zeolite W, disclosed in U.S. Patent No. 3 o12 853 to R.M. Milton, has been known to be selective to ammonia and has been used in waste-water treatment. It has been found that particular zeolite W compositions will sorb ammonia at the lower concentrations found in dialysate, and also will provide for the removal of potassium and the release of calcium in the right

proportion. The compositions of the invention are suitable for applications involving ammonia concentrations in the dialysate as low as 1 meq/l.

The composition of zeolite W can be expressed in terms of oxides of the components as:

$$1.0 \pm 0.1\ M_{2/n}O : Al_2O_3 : 4.1 \pm 0.8\ SiO_2 : X\ H_2O,$$

where M is an exchangeable cation, n is the valence of M and X is about 5.1 in the fully hydrated form. Like most zeolites, zeolite W consists of a network of $AlO_4$ and $SiO_4$ tetrahedra joined in a lattice with adjacent tetrahedra sharing 2 oxygen atoms. The alumina tetrahedra have a net charge of -1 which is balanced by the presence of a nearby exchangeable cation. The alumina tetrahedra, therefore, provide for cation sites that are occupied by the exchangeable cations. Compositions of zeolite W can be prepared where there is more than one species of exchangeable cation M, such as a composition containing sodium, potassium and calcium cations. When particular zeolite W compositions of the invention containing those three cations as exchangeable cations are contacted with a solution containing sodium, potassium, calcium and ammonium cations of concentrations typical of a dialysate in a slurried system, the following reactions occur:

$$(5)\quad 2NH_4^+ + (Ca)Z \longrightarrow Ca^{+2} + (2NH_4^+)Z$$

$$(6)\quad K^+ + (Na)Z \longrightarrow Na^+ + (K)Z$$

where Z represents zeolite W and the parentheses denote the exchangeable cations. The dynamics of the system are such that essentially one milli-equivalent of sodium cations is ion-exchanged for one milli-equivalent of potassium cations; and essentially one milli-equivalent of calcium is ion-exchanged for one milli-equivalent of

ammonium. This property allows the release of calcium and the removal of potassium in a set proportion to each other.

This can be explained by reference to Figure 1. Figure 1 is a ternary diagram commonly used to show compositions of three component systems. It represents initial compositions of zeolite W as defined by areas CDGF and CDE which compositions are suitable for use in a slurried dialysate. By "initial" is meant the zeolite composition before it is contacted with the dialysate solution. The axes of Figure 1 are equivalent-percent of calcium cations, equivalent-percent of potassium cations and the equivalent-percent of sodium and ammonium cations combined. The basis of the percentages is the loadings of calcium, potassium, sodium and ammonium only. It is contemplated that other cations, principally magnesium and hydrogen, will also occupy some other cation sites. The magnesium loading should be about 0 to 2 equivalent percent based on the total number of cation sites. In general, magnesium cations should not be added to the dialysate during treatment, some removal being desirable. At the indicated level of magnesium on the zeolite, there will be no significant net ion-exchange of magnesium into the dialysate; a small amount will be removed from the dialysate at the lower indicated levels.

It is also contemplated that some of the cation-exchange sites may be occupied by "hydrogen cations", which are exchangeable in a similar fashion to other cations, although they occupy the cation sites in a different manner. The level of "hydrogen cations" should be between 4 and 11 equivalent-percent in the initial zeolite composition, based on the total number of cation sites.

Some "hydrogen cations" on the zeolite are desirable to buffer the dialysate against the carbonate-bicarbonate reaction (3) and help maintain the pH level in the blood at normal physiological levels.

Although calcium, potassium, sodium and ammonium are the principal exchangeable cations, exchangeable cations on the zeolite other than calcium, potassium, sodium and ammonium will also ion exchange with cations in the dialysate solution; but if the percentages of the magnesium cations and "hydrogen cations" are within the indicated limits and the percentage of other cations is negligible, there will be little effect on the ion-exchange properties in relation to calcium, potassium, sodium and ammonium as shown in reactions (5) and (6) and as discussed in this specification. Therefore, the zeolite W compositions of the invention can be described using only a calcium, potassium, sodium and ammonium basis.

The calcium levels shown in Figure 1 are based on "free-calcium" or calcium that can ion-exchange. In the presence of carbonate, some calcium in the form of calcium carbonate will exist on or in the zeolite and not be ion-exchangeable. In an analysis of the zeolite, the amount of "free-calcium" can be found by subtracting the equivalents of carbonate from the equivalents of total calcium present in the zeolite.

Referring to Figure 1, point E represents the composition of zeolite W in equilibrium with a dialysate solution of typical composition and also containing ammonia as if in a slurried dialysate system. Equilibrium values were derived by contacting samples of zeolite W with commercial dialysate solution samples, with and without ammonia, for several hours until equilibrium was reached, and then

analyzing the zeolite for its composition. Point E on Figure 1 represents the equilibrium composition with a commercial dialysate containing 3 meq/l of ammonia. At the end of a dialysis treatment the composition of the zeolite would be near equilibrium with the dialysate which point is represented by point E on Figure 1. The composition of the commercial dialysate used (excluding the added ammonia) is shown in Table A.

Table A

| | Concentration (meq/l) |
|---|---|
| Sodium | 134 |
| Calcium | 3.5 |
| Potassium | 2.o |
| Magnesium | 1.5 |
| Chloride | 1o4.4 |
| Acetate | 36.6 |
| Dextrose | 2.5 grams/l |

The commercial dialysis solution shown in Table A is generally used in a system wherein the dialysis is not renewed but wherein a fresh dialysate solution is continuously passed over the semi-permeable membrane. In the system contemplated by the invention, the composition of the dialysate would remain substantially the same for most ions throughout the treatment even though it is repeatedly contacted with the membrane. Sodium may be added or removed from the dialysate solution due to the ion-exchange of cations on the zeolite as explained elsewhere. Therefore, depending on the initial zeolite composition used, sodium will be added or removed from the dialysate. The sodium balances

can be adjusted by methods known in the art, such as by dietary restraints, adjustment of ultrafiltrate water removed during treatment, or adjustment of $Na^+$ levels in the initial dialysate solution. The main mechanism of maintaining the cation balance is through the ion-exchange properties of the zeolite, compensating for the addition and removal of the potassium and calcium constituents of the dialysate due to chemical reaction and diffusion across the membrane. In this manner the dialysate ist renewed and maintained in a closed system. With a single composition, it is possible to provide for adequate ammonium selectivity and also provide for both the proper calcium release and potassium removal. The composition at point E is 11.5 equivalent-percent calcium, 2o.8 equivalent-percent potassium, 47.2 equivalent-percent sodium, and 2o.5 equivalent-percent ammonium. For purposes of showing point E on Figure 1, the concentration of sodium and ammonia are considered together as one component at 67.7 equivalent-percent. This was done to enable representation in two dimensions. Calcium and potassium are shown singly so that derivation of the compositions of the invention can be shown from the calcium-release to potassium-removal ratios, which are represented by straight lines on Figure 1.

Lines AE and BE are the lines of constant slope 5:1 and 12:1, respectively, where the slope is defined as (+ equivalents $Ca^{+2}$/-equivalents $K^+$). If an initial zeolite W composition is represented by a point on line AE, during a dialysis treatment using a slurried dialysate the composition of the zeolite will move towards point E, releasing 5 equivalents of calcium to one equivalent of potassium removed. Likewise the ratio would be 12:1 for a composition represented by a point

on line BE. The preferred range of ratios of calcium release to potassium removal are therefore represented by a family of tie lines emanating from point E and existing between lines EA and EB. The initial compositions represented by the area EAB will provide for the desired ratios of calcium addition to potassium removal. Lines EC and ED correspond to ratios of 4:1 to 16:1, respectively, and therefore area ECD represents compositions which will provide for suitable ratios for dialysis applications, of calcium release to potassium removal, including those where there is to be a special regulation of calium or potassium in the system. It is understood that these initial compositions contain little or no ammonia. The initial compositions of the invention, as represented by Figure 1, contain calcium, potassium and sodium only as the principal exchangeable cations.

In providing for the proper calcium-potassium ratio or proportion, the composition of zeolite W is limited by the ammonia capacity and calcium loading of the zeolite. Reaction (5) can occur only to the extent of the capacity of the zeolite for ammonia. If equation (5) is to occur during the entire dialysis process and therefore the calcium-potassium ratio be nearly constant over the course of the dialysis process, the equivalents of calcium released cannot exceed equivalents of ammonia removed. Therefore, the available capacity for ammonia at equilibrium with the dialysate solution provides a limit for the calcium loading if the calcium-potassium ratio is to remain set over the course of the process. This upper limit of calcium for compositions to be used with a dialysate containing 3.o meq/l of ammonia is represented by line CD on Figure 1. For initial compositions, represented by area CDE of Figure 1, the

calcium-potassium ratios will be constant as discussed above; however, for compositions having excess calcium that are represented by points above line CD, the equivalents of ammonia (and urea) removed will be exceeded by the amount of calcium released. Therefore, there will be a net addition of calcium cations to the dialysate solution, the calcium release exceeding that withdrawn from the solution in the form $CaCO_{3(s)}$. A net addition of calcium may be desirable for some patients with calcium deficiency or other problems or to control anions in the dialysate such as phosphate. So, compositions with excess calcium that provide for a net addition of calcium cations may be suitable compositions for dialysis. However, it is undesirable that in providing for a net addition of calcium cations that additional potassium be removed or added. The potassium removal should remain in set proportion to the calcium reacted that forms $CaCO_{3(s)}$ in reaction (3). Therefore, the properties of the zeolite should provide that the equivalents of calcium corresponding to the amount of excess calcium be ion-exchanged with sodium cations in the dialysate, rather than potassium cations. This can be accomplished by providing that the potassium loadings of compositions with excess calcium have the same potassium loadings as the corresponding compositions represented by line CD. Referring to Figure 1, point C on line CD represents a composition that provides for a calcium-potassium ratio of 4:1 where there is no net calcium cation addition. Line CF represents compositions, corresponding to point C, wherein the ratio of equivalents of calcium released in reaction (5) to the equivalents of potassium removed in equation (6) equals 4:1, and wherein additional calcium cations have been added to

the dialysate solution. In a similar fashion, other lines representing other excess calcium compositions corresponding to other calcium-potassium ratios can be found, e.g., line DG which corresponds to a ratio of 16:1. Area CDGF represents the excess calcium compositions suitable for dialysis that provide for a net calcium cation addition. Area ECD represents compositions suitable for dialysis, wherein no net calcium cation addition is provided.

The compositions represented by Figure 1 are compositions suitable for use in a dialysate containing 3 meq/l ammonia. It may be desirable to have the ammonium concentration at a lower level within the range of 1-3 meq/l. For a desired concentration, an equilibrium point can be found and suitable initial compositions can be derived in the same manner as shown above. For each equilibrium point, the suitable compositions will be represented by an area similar to that of area ECFGD of Figure 1. Figure 2 is a ternary diagram representing zeolite W compositions on the same basis as in Figure 1. Referring to Figure 2, the line HI represents the equilibrium compositions of zeolite W for concentrations of from 1-3 meq/l of ammonia in the dialysate. Point H represents the composition corresponding to a concentration of 3 meq/l of ammonia and is the same as point E on Figure 1. Point I represents the composition corresponding to a concentration of 1 meq/l of ammonia. The position of the other points can be found by linear interpolation, the relevant equilibrium relationship being nearly linear in this range. From these points, overlapping areas representing suitable dialysis compositions can be derived. Area HIJKLM encompasses all these areas

and represents, therefore, all the compositions of the invention suitable for kidney dialysis. The data used to derive area HIJKLM are summarized in Tables B and C. Table B shows the position of the equilibrium points representing the equilibrium compositions in equivalent-percent for various ammonia concentrations in dialysate. Also shown are the labels of the corresponding points (c.p.) on Figures 1 and 2. Table C shows the compositions in equivalent-percent corresponding to the upper limit of calcium for various calcium-potassium ratios and ammonia concentrations in the dialysate. The corresponding points on Figures 1 and 2 are also shown.

Table B

| $NH_4^+$ in dialysate (meq/l) | $Ca^{+2}$ | $K^+$ | $NH_4^+$ | $Na^+$ | c.p. |
|---|---|---|---|---|---|
| 1.o | 12.8 | 25.8 | 6.8 | 54.6 | I |
| 2.o | 12.1 | 23.3 | 13.7 | 5o.9 | |
| 3.o | 11.5 | 2o.8 | 2o.5 | 47.2 | E,H |

Table C

| $NH_4^+$ in dialysate (meq/l) | $-Ca^{+2}/K^+$ | $Ca^{+2}$ | $K^+$ | $Na^+ + NH_4^+$ | c.p. |
|---|---|---|---|---|---|
| 1.o | 4:1 | 19.6 | 24.1 | 56.3 | |
| | 5:1 | 19.6 | 24.4 | 56.o | |
| | 12:1 | 19.6 | 25.2 | 55.2 | |
| | 16:1 | 19.6 | 25.4 | 55.o | J |
| 2.o | 4:1 | 25.8 | 19.9 | 54.3 | |
| | 5:1 | 25.8 | 2o.6 | 53.6 | |
| | 12:1 | 25.8 | 22.2 | 52.o | |
| | 15:1 | 25.8 | 22.4 | 51.8 | |
| 3.o | 4:1 | 32.1 | 15.7 | 52.2 | M,C |
| | 5:1 | 32.1 | 16.7 | 51.2 | A |
| | 12:1 | 32.1 | 19.1 | 48.8 | B |
| | 16:1 | 32.1 | 19.5 | 48.4 | D |

Thus area HIJKLM in Figure 2 is defined by the area enclosed by lines connecting points corresponding to compositions in equivalent-percent as shown below in Table D.

Table D

| Point | $Ca^{+2}$ | $K^{+}$ | $Na^{+}$ + $NH_4^{+}$ |
|---|---|---|---|
| H | 11.5 | 2o.8 | 67.7 |
| I | 12.8 | 25.8 | 61.4 |
| J | 19.6 | 25.4 | 55.o |
| K | 74.6 | 25.4 | 0 |
| L | 84.3 | 15.7 | 0 |
| M | 32.1 | 15.7 | 52.2 |

Thus, the present invention provides for the simultaneous regulation of calcium and potassium where there is to be a net addition of calcium cations and also where there is no net addition of calcium cations to the system.

The invention also inlcudes compositions suitable for use in a slurried dialysate, which compositions comprise zeolite W, wherein the initial compositions are represented by the area HIJKLM in Figure 2.

Also included is a process for hemodialysis using a slurried dialysate containing an ion-exchange composition, said composition being a composition as described above.

The slurried dialysate will contain other constituents other than the zeolite W. For example, it is also contemplated that a suspending agent will be necessary to maintain any solid phase, such as the zeolite, in suspension in a slurry. The urease enzyme may also be present in the dialysate supported on a solid surface such as the zeolite or other solid

support or be in a separate compartment or provided in any other method which contacts the dialysate with the enzyme. The zeolite, suspending agent, possibly the urease enzyme, and water are the principle constituents of the slurried dialysate that is contemplated by the invention. However, it is contemplated that other substances may be desired to sorb other uremic substances or to improve the rheological or sorptive properties of the slurried dialysate.

The zeolite W compositions of the invention are suitable as dialysate compositions because of their unique combination of selectivity and capacity towards ammonia and their ion-exchange properties relating to potassium and calcium. Other compositions of zeolite W and other zeolites have been suggested in the prior art as suitable; however, none have shown a simultaneous regulation of calcium and potassium along with good ammonia selectivity and capacity. Most zeolite compositions have properties unsuitable for applications in a slurried dialysate system, due to selectivity towards ammonia or unsuitable properties relating to calcium and potassium. As an example, a zeolite W having no potassium as exchangeable cations can remove too much potassium from the dialysate if the dialysate is allowed to approach equilibrium with the blood. Such a zeolite composition, even though it may exhibit a favorable selectivity and capacity for ammonia, if it does not properly regulate the calcium or potassium concentrations, is unsuitable. Compositions of zeolites other than zeolite W may be suitable, but these other zeolites will necessarily have different equilibrium

properties and selectivities. Therefore, such compositions will not necessarily correspond to those of zeolite W as shown in Figure 2. In order to establish that compositions of a particular zeolite are suitable, the equilibrium properties and kinetics of the zeolite must be analyzed to establish its suitability for use in slurried dialysate systems and also to establish the composition boundaries, as was shown in the case for zeolite W in Figur 1. This analysis must be done for each zeolite species, owing to the different properties of each.

What is claimed is:

1. A hemodialysis composition comprising zeolite W in a slurried dialysate solution wherein the amount of exchangeable ions in the zeolite are within the following ranges:

   a) around 11.5 to 85 equivalent-percent calcium ions,
   b) around 15.7 to 25.4 equivalent-percent potassium ions,
   c) around 0 to 56.3 equivalent-percent sodium ions, and
   d) around 0 to 2o.5 equivalent-percent ammonium ions.

2. The composition of claim 1 wherein the dialysate solution has an ammonium concentration of within the range of around 1 to 3 milli-equivalents per liter of solution.

3. The composition of claim 1 wherein the dialysate solution contains a suspending agent, urease enzyme and water.

4. Compositions suitable for use in a slurried dialysate, which compositions comprise zeolite W, wherein the initial, compositions are represented by the area HIJKLM in Figure 2 of the specification.

5. A process for hemodialysis using a slurried dialysate solution containing an ion-exchange

composition which comprises zeolite W wherein the amount of exchangeable ions in the zeolite are within the following ranges:

a) around 11.5 to 85 equivalent-percent calcium ions,
b) around 15.7 to 25.4 equivalent-percent potassium ions,
c) around 0 to 56.3 equivalent-percent sodium ions, and
d) around 0 to 2o.5 equivalent-percent ammonium ions.

6. A process for hemodialysis using a slurried dialysate containing an ion-exchange composition, said composition being a composition comprising zeolite W, wherein the initial composition is represented within the area HIJKLM in Figure 2 of the specification.

7. A process for removing urea from blood comprising the steps of:
1) contacting the blood with a slurried dialysate solution containing a urease enzyme which hydrolizes urea into ammonium ions; and
2) removing the ammonium ions from the solution by adsorption onto a zeolite W composition in the dialysate solution.

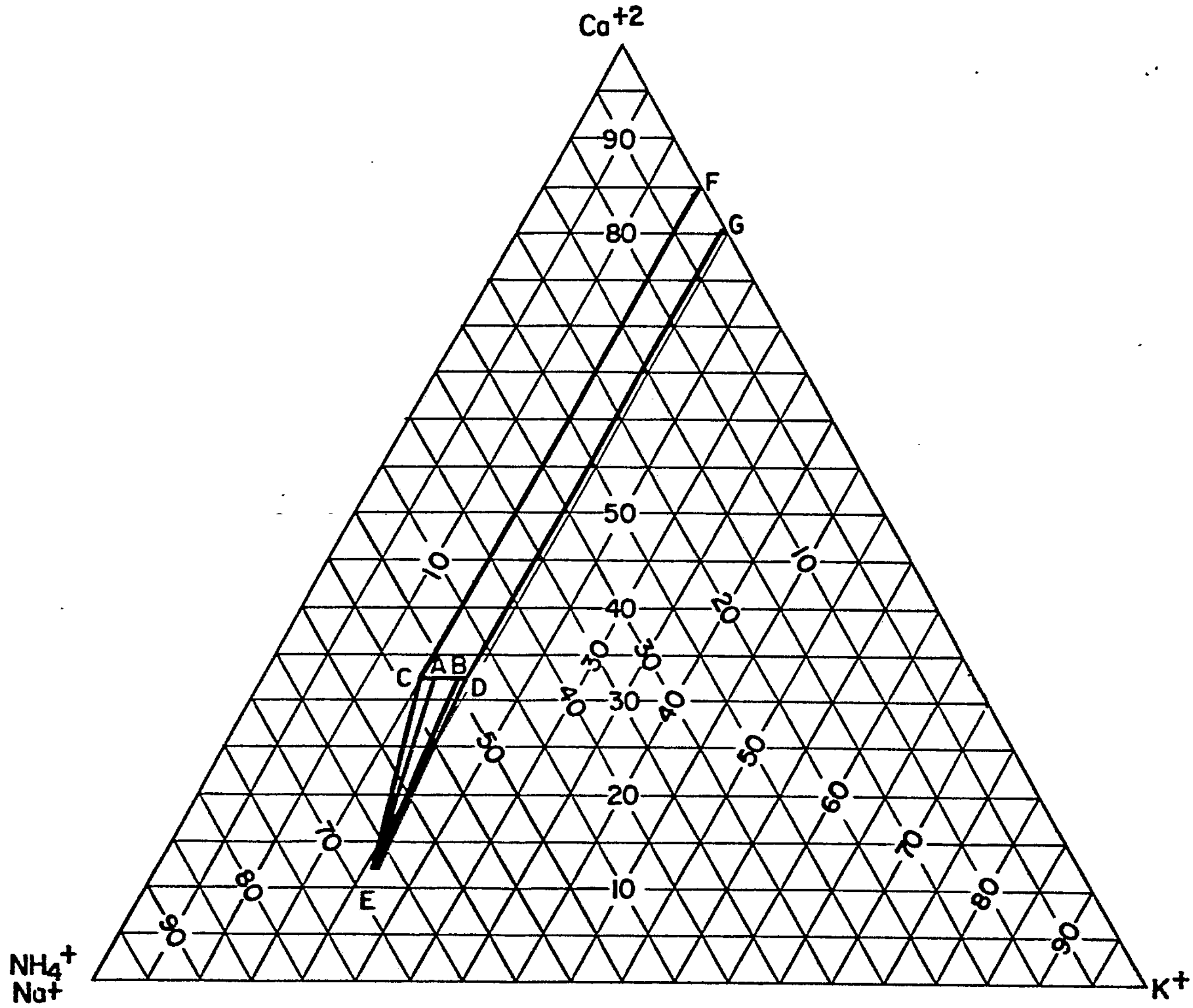
Ca+2
90
80
50
40
30
20
10
F
G
A
B
C
D
E
10
20
30
40
50
60
70
80
90
10
30
40
50
70
80
90
NH4+
Na+
K+

FIG. 1

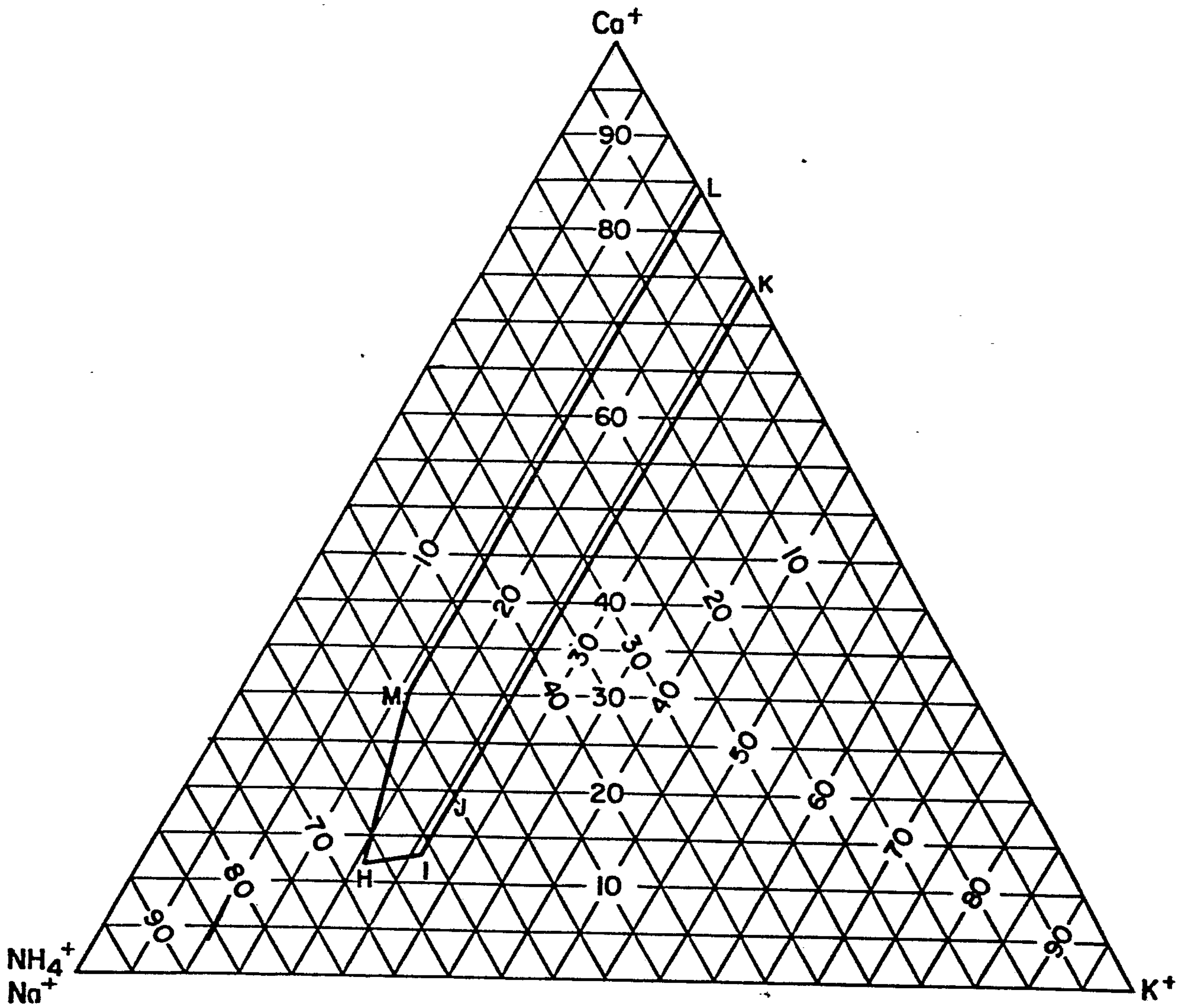
Ca+
90
80
L
K
60
10
20
40
30
30
40
30
40
20
10
20
M
50
60
J
20
70
H
I
10
70
80
80
90
90
NH4+
Na+
K+

FIG. 2

0046971

European Patent Office

# EUROPEAN SEARCH REPORT

Application number
EP 81 10 6583

## DOCUMENTS CONSIDERED TO BE RELEVANT

| Category | Citation of document with indication, where appropriate, of relevant passages | Relevant to claim |
| --- | --- | --- |
| | EP - A - 0 003 914 (PURDUE RES. FOUND.)<br>* The whole document *<br>-- | 1-7 |
| A | FR - A - 2 264 586 (GAMBRO)<br>* Page 15, claims * | 1,3,5, 7 |
| D | & GB - A - 1 484 642<br>-- | |
| AD | US - A - 4 071 444 (ASH) | |
| A | US - A - 3 463 728 (KOLOBOW) | |
| A | US - A - 3 669 878 (MARANTZ) | |
| A | DE - B - 2 152 355 (SARTONIUS-MEMBRAN-FILTER) | |
| A | FR - A - 2 278 634 (UNION CARBIDE)<br>---- | |

CLASSIFICATION OF THE APPLICATION (Int. Cl.3)

A 61 M 1/03
B 01 J 39/02

TECHNICAL FIELDS SEARCHED (Int. Cl.3)

A 61 M 1/03
B 01 J 39/02

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
| --- | --- | --- |
| The Hague | 01-12-1981 | WENDLING |

EPO Form 1503.1 06.78